# EUROPEAN PATENT APPLICATION

(11) **EP 2 266 559 A1**
(43) Date of publication of application: **29.12.2010**
(21) Application number: 10177395.0
(22) Date of filing: 14.03.2006
(51) Int. Cl.: A61K 31/20, A61K 45/00, A61P 27/02, A61P 27/06, A61P 27/10, A61P 27/12, A61P 43/00

(54) **Therapeutic agent for ophthalmic disease**

(30) Priority: 15.03.2005 JP 2005072837
(62) Divisional of application: 09156822.0
(71) Applicant: ONO PHARMACEUTICAL CO., LTD., Osaka-shi, Osaka 541-8526 (JP)
(72) Inventor: Maegawa, Hitoshi, Osaka 618-8585 (JP)
(74) Representative: Schön, Christoph

(57) **Abstract**

The present invention provides an agent comprising (2R)-2-propyloctanoic acid or a salt thereof for use in the prevention, treatment and/or inhibition of symptom progression of subject of an ophthalmic disease selected from the group consisting of glaucoma, cataract, retinal detachment, muscae volitantes, age-related macular degeneration, diabetic retinopathy, macular edema, myopia, asthenopia, choked disc, papillitis optica, retrobulbar neuritis, toxic amblyopia, optic atrophy, higher visual pathway lesion, internuclear ophthalmoplegia, gaze palsy and ischemic optic neuropathy, wherein the agent is administered intravenously for from one day to 7 days at a dose of from 2 mg to 12 mg per 1 kg of body weight of a patient, orally in an amount per dose of from 300 mg to 1200 mg for from one day to five years or by ocular instillation in an amount per dose of from 0.3 ng to 1 mg for from one day to one year.

## Description

### TECHNICAL FIELD

The present invention relates to an agent for prevention, treatment and/or inhibition of symptom progression of an ophthalmic disease and an agent for optic nerve protection, each of which comprises (2R)-2-propyloctanoic acid, a salt thereof or a prodrug thereof, in which an amount per dose is from 0.3 ng to 5000 mg.

### BACKGROUND ART

The number of patients with glaucoma, which is a disease inducing ophthalmic dysfunctions such as, iris adhesion, lens bulging, and the like and organ disorders (retinal nerve degeneration and necrosis), has been continuously increasing with the growth of the aged population and serious eye strain such as caused by a computer, and the like. Although it is said that an increase in ocular tension is a causative factor of glaucoma, the incidence thereof is not so low in those having normal ocular tension. Since retinal nerve damage can be hardly restored by the currently available medical techniques, early detection and treatment are fundamentally required. As treatment of glaucoma, pharmacotherapy such as ocular instillation of agents for lowering ocular tension has been carried out, The agents for lowering ocular tension include those inhibiting production of aqueous humor and those promoting aqueous humor drainage. As the agents belonging to the former group, β-blocking agents, carbonic anhydrase inhibitors (CAI) and the like have been used, while parasympathetic agonists, α1-blocking agents, prostaglandins and the like have been used as the agents belonging to the latter group. In the case where any sufficient effect cannot be obtained by the pharmacotherapy, a surgical operation is carried out. The surgical operation method carried out in these days is that the aqueous humor is drained into the subconjunctival space via an artificially formed drainage channel to lower ocular tension. However, the method induces postoperative adhesion or scarring of the subconjunctival tissue and thus the aqueous humor cannot be discharged, which sometimes results in an increase in ocular tension against the original intention. Actually, it is reported that the rate of procedural success during the postoperative 5 years is about 50 to 60% (Graefe. Arch. Clin. Exp. Ophthalmol., (2003)). Since restore of the nerve damage cannot be expected by these pharmacotherapy or surgical operation as discussed above, it has been required to establish a method which is effective even to a patient in the advanced stage of glaucomatous nerve damage.

On the other hand, it has been reported that (2R)-2-propyloctanoic acid, which has an activity of reducing the intracellular S-100β content, has a potentiality to be used as an agent for the treatment or prevention of various cranial nerve diseases including stroke, through the improvement of the function of abnormally activated astrocyte (*e*.*g*., see Journal of Cerebral Blood Flow & Metabolism, 22(6), 723-734 (2002) (non-Patent Document 1)).

Also, since pentanoic acid derivatives including (2R)-2-propyloctanoic acid have an action to improve functions of astrocyte, it is known that they are useful as a brain function improving agent and are effective for treating Alzheimer's disease, amyotrophic lateral sclerosis and neurological dysfunction of stroke or brain injury. In addition, it has been described that they are orally administered once to several times a day within a range of 1 to 1,000 mg per one dose per adult, or parenterally administered once to several times a day within a range of 0.1 to 100 mg per one dose (*e*.*g*., see the specification of European Patent No. 0632008 (Patent Document 1)).

Moreover, as documents reporting the relationship between (2R)-2-propyloctanoic acid and ophthalmic diseases, the followings are known. For example, WO 03/020281 (Patent Document 2) discloses that ONO-2506, namely (2R)-2-propyloctanoic acid can be used for treating dry eye based on optic nerve disorder; and WO 00/032197 (Patent Document 3) discloses that (2R)-2-propyloctanoic acid can be used as a neurotrophic factor stimulant in treating glaucoma and optic papilla neurosis. However, in the specifications of the Patent Documents 2 and 3, only AIT-082 (neotrophin) related matted is disclosed. In these documents, description of (2R)-2-propyloctanoic acid is only as "ONO-2506" and even its structure is not disclosed.
Non-Patent Document 1 Journal of Cerebral Blood Flow & Metabolism, 22(6), 723-734 (2002)
Patent Document 1 EP 0632008
Patent Document 2 WO 03/020281
Patent Document 3 WO 00/032197

### DISCLOSURE OF THE INVENTION

### PROBLEMS TO BE SOLVED BY THE INVENTION

As described above, Patent Documents 2 and 3 do not disclose specific description about ONO-2506, namely, (2R)-2-propyloctanoic acid. Thus, these documents neither disclose nor suggest the dose and an administration method which is appropriate for achieving a therapeutic effect on ophthalmic disease.

Accordingly, an object of the present invention is to provide a specific method of administering (2R)-2-propyloctanoic acid for treating the diseases described in Patent Documents 2 and 3 such as glaucoma, optic papilla neurosis and dry eye and a specific method of administering the same for treating other ophthalmic diseases. Namely, it is intended to provide a treatment method which is effective applicable even to a patient in the advanced stage of glaucomatous nerve damage who cannot be ameliorated by the known treatment methods.

### MEANS FOR SOLVING THE PROBLEMS

The inventors of the present invention studied the effects of (2R)-2-propyloctanoic acid on rat models of retinal ischemia. As a result, they found out that the intravenous administration of (2R)-2-propyloctanoic acid at the dose of 10 mg/kg once a day for 7 days after the revascularization of the ischemia has remarkable effect of inhibiting optic nerve cell death induced by the retinal ischemia. Based on the finding, the inventors of the present invention have conducted further studies and accomplished the present inventions.

Thus, the present invention relates to :
[1] an agent for prevention, treatment and/or inhibition of symptom progression of an ophthalmic disease comprising (2R)-2-propyloctanoic acid, a salt thereof or a prodrug thereof, in which an amount per dose is from 0.3 ng to 5000 mg ;
[2] the agent according to above [1], which is for oral administration, intravenous administration or ocular instillation ;
[3] the agent according to above [2], wherein the amount per dose in the oral administration is from 100 mg to 5000 mg ;
[4] the agent according to above [2], wherein the amount per dose in the intravenous administration is from 100 mg to 1200 mg ;
[5] the agent according to above [4], wherein the dose per 1 kg of body weight of a patient is from 2 mg to 12 mg ;
[6] the agent according to above [2], wherein the amount per dose in the ocular instillation is from 0.3 ng to 1 mg ;
[7] the agent according to above [1], which is the agent for prevention, treatment and/or inhibition of symptom progression of glaucoma, cataract, retinal detachment, muscae volitantes, age-related macular degeneration, diabetic retinopathy, macular edema, myopia, asthenopia, dry eye, amaurosis fugax, choked disc, papillitis optica, retrobulbar neuritis, toxic amblyopia, optic atrophy, higher visual pathway lesion, internuclear ophthalmoplegia, gaze palsy or ischemic optic neuropathy ;
[8] the agent according to above [1], which further comprises one or more selected from the group consisting of sympathomimetic agents, parasympathomimetic agents, sympathetic blockers, prostaglandins, carbonic anhydrase inhibitors and hypertonic osmotic agents ;
[9] the agent according to above [1], which is the agent for optic nerve protection ;
[10] a method for prevention, treatment and/or inhibition of symptom progression of an ophthalmic disease comprising administration of (2R)-2-propyloctanoic acid, a salt thereof or a prodrug thereof to a mammal, in which an amount per dose is from 0.3 ng to 5000 mg ;
[11] a method for optic nerve protection comprising administration of (2R)-2-propyloctanoic acid, a salt thereof or a prodrug thereof to a mammal, in which an amount per dose is from 0.3 ng to 5000 mg ;
[12] use of (2R)-2-propyloctanoic acid, a salt thereof or a prodrug thereof for the manufacture of an agent for prevention, treatment and/or inhibition of symptom progression of an ophthalmic disease comprising (2R)-2-propyloctanoic acid, a salt thereof or a prodrug thereof, in which an amount per dose is from 0.3 ng to 5000 mg ;
[13] use of (2R)-2-propyloctanoic acid, a salt thereof or a prodrug thereof for the manufacture of an agent for optic nerve protection comprising (2R)-2-propyloctanoic acid, a salt thereof or a prodrug thereof, in which an amount per dose is from 0.3 ng to 5000 mg ;
   and so forth.

### EFFECT OF THE INVENTION

According to the present invention, it is possible to provide a specific method of administering (2R)-2-propyloctanoic acid, a salt thereof or a prodrug thereof for treating an ophthalmic disease such as glaucoma, optic papilla neurosis or dry eye and a specific method of administering the same for treating other ophthalmic diseases. By administering (2R)-2-propyloctanoic acid, a salt thereof or a prodrug thereof with a preferable administration method and a preferable dose which are disclosed by the present invention, it is possible to achieve effects of protecting optic nerve, such as preventing optic nerve cell death which is described hereinafter. Accordingly, the present invention provides a treatment method which is effective even to a patient in the advanced stage of glaucomatous nerve damage that cannot be ameliorated by the currently available treatment methods.

### BEST MODE FOR CARRYING OUT THE INVENTION

In the present invention, (2R)-2-propyloctanoic acid is a compound represented by formula (1) : 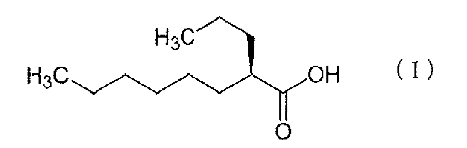 wherein 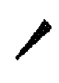 represents β-confuguration.

In the present invention, the salt of (2R)-2-propyloctanoic acid is preferably a pharmaceutically acceptable salt. The pharmaceutically acceptable salt is preferably a non-toxic water-soluble salt. Examples of suitable salt of (2R)-2-propyloctanoic acid, for example, include salts with inorganic bases, salts with organic bases, salts with basic natural amino acids and so forth. The salt with an inorganic base is preferably, for example, an alkali metal salt (*e*.*g*., a sodium salt, a potassium salt, a lithium salt, *etc*.), an ammonium salt (*e*.*g*., a tetramethylammonium salt, a tetrabutylammonium salt, *etc*.), or the like, The salt with an organic base is preferably, for example, a salt with alkylamine (*e.g.*, methylamine, dimethylamine, trimethylamine, triethylamine, *etc*.), heterocyclic amine (*e*.*g*., pyridine, picoline, piperidine, *etc*.), alkanolamine (*e.g.*, ethanolamine, diethanolamine, triethanolamine, *etc.*), dicyclohexylamine, N,N'-dibenzylethylenediamine, cyclopentylamine, benzylamine, dibenzylamine, phenethylamine, tris(hydroxymethyl)methylamine, N-methyl-D-glucamine or the like. The salt with a basic natural amino acid is not particularly limited, so long as it is a salt with a basic amino acid which is naturally present and can be purified, and it is preferably, for example, a salt with arginine, lysine, ornithine, histidine or the like.

(2R)-2-Propyloctanoic acid or a salt thereof can be prepared by methods known *per se,* for example, the methods described in the specification of EP 0632008, the specification of WO 99/58513, the specification of WO 00/48982, the specification of JP 3032447 (registration number), the specification of JP 3084345 (registration number), the specification of WO 03/051852, the specification of WO 04/110972, similar method thereof, or the method described in Comprehensive Organic Transformations : A Guide to Functional Group Preparations, 2nd Edition (Richard C. Larock, John Wiley & Sons Inc., 1999), or by appropriate combinations of such methods. The reaction product may be purified by conventional purification means, for example, by distillation at atmospheric or reduced pressure, high performance liquid chromatography, thin layer chromatography or column chromatography using silica gel or magnesium silicate, or by conventional methods such as washing and recrystallization. Also, if necessary, the product may be subjected to treatments such as lyophilization.

In the present invention, a structure of a prodrug of (2R)-2-propyloctanoic acid is not particularly limited so long as it is a (2R)-2-propyloctanoic acid derivative which can be enzymatically or chemically converted into (2R)-2-propyloctanoic acid *in vivo.* Examples of the prodrug of (2R)-2-propyloctanoic acid include a compound in which a carboxyl group has been esterified (*e.g.*, methyl ester, ethyl ester, isopropyl ester, isobutyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester or cyclohexyloxycarbonylethyl ester of (2R)-2-propyloctanoic acid, *etc.*), a compound in which a carboxyl group has been amidated (*e.g.*, methylamide, ethylamide or phenylamide of (2R)-2-propyloetannic acid, *etc.),* an alcohol compound in which a carboxy group has been reduced or a protected compound thereof (*e.g.*, (2R)-2-propyloctanol, (2R)-2-propyloctanyl ester of acetic acid, *etc*.), and so on. In the case of using such a prodrug in ocular instillation, it is particularly preferable, as will be described hereinafter, that a prodrug of (2R)-2-propyloctanoic acid is a fat-soluble ester such as a C1 to C5 alkyl ester of (2R)-2-propyloctanoic acid (*e*.*g*., methyl ester, ethyl ester, isopropyl ester, isobutyl ester, *etc*.). These compounds can be produced by publicly known methods. Also, a prodrug of (2R)-2-propyloctanoic acid may be a compound which is converted into (2R)-2-propyloctanoic acid under physiological conditions as described in lyakuhin no Kaihatsu, vol.7, Bunshi Sekkei, p.163-198 (Hirokawa Shoten, 1990). Furthermore, a prodrug of (2R)-2-propyloctanoic acid may be a salt or a solvate as described above and it may be labeled with an isotope (*e*.*g.*, ³H, ¹⁴C, ³⁵S, ¹²⁵I, *etc*.).

(2R)-2-propyloctanoic acid, a salt thereof or a prodrug thereof is not limited to those which are substantially pure single substances, and they may include impurities (*e*.*g*., byproducts, solvents, starting materials, *etc.* which originate from the preparation process, or decomposition products, *etc*.) within the scope acceptable in an active ingredient for a medicament. The content of impurities acceptable as the medicinal bulk varies depending on whether the (2R)-2-propyloctanoic acid is used, a salt thereof is used or a prodrug thereof is used, and also varies depending on the contained impurities, but in the case of the (2R)-2-propyloctanoic acid, for example, it is desirable that heavy metals are about 20 ppm or less, the S-form as its optical isomer is about 1.49% by mass or less, 2-propanol and heptane as the residual solvents are about 5000 ppm or less in total, and the moisture is about 0.2% by mass or less. Particularly, (2R)-2-propyloctanoic acid having an optical purity of about 99% e.e. or more, more particularly, (2R)-2-propyloctanoic acid having an optical purity of about 99.3% e.e. or more, is suitable.

To prevent, treat and/or inhibit of symptom progression of an ophthalmic disease, the present invention discloses a method which comprises administering an effective amount namely, from about 0.3 ng to about 5000 mg per dose of (2R)-2-propyloctanoic acid, a salt thereof or a prodrug thereof to a mammal (a human being is preferable and a patient is more preferable). In the method, it is particularly preferable to use a free compound, namely, (2R)-2-propyloctanoic acid. In the case of using a salt or a prodrug of (2R)-2-propyloctanoic acid, although it may be used either in an amount of from about 0.3 ng to about 5000 mg per dose as the salt or prodrug of (2R)-2-propyloctanoic acid or in an amount of from about 0.3 ng to about 5000 mg per dose in terms of (2R)-2-propyloctanoic acid, though it is preferable to administer the salt or prodrug in an amount of from about 0.3 ng to about 5000 mg per dose in terms of (2R)-2-propyloctanoic acid. In the present invention, the term "prevention" means to prevent the onset of disease itself; "treatment" means to lead the state of disease to cure; and "suppression of progress" means to suppress the deterioration or stop the progress of the state.

In the present invention, the ophthalmic disease is not particularly limieted so long as it is a disease causing a disorder affecting eye and/or visual sense. Examples thereof include, for example, orbital disorders (*e.g.*, orbital cellulitis, cavernous sinus thrombosis, exophthalmos, *etc*.), lacrimal apparatus disorders (*e*.*g*., lacrimal passage stenosis, dacryocystitis, *etc.*), eyelid disorders (*e.g.*, eyelid edema, blepharitis, hordeolum, chalazion, entropion, ectropion, tumor, *etc*.), conjunctival diseases [*e.g.*, acute conjunctivitis, viral conjunctivitis, nongonococcal bacterial conjunctivitis, adult gonococcal conjunctivitis, inclusion body conjunctivitis (*e*.*g*., adult inclusion body conjunctivitis, newborn inclusion body conjunctivitis, inclusion body hyperacute purulent conjunctivitis, swimming pool conjunctivitis, *etc.*), seasonal allergic conjunctivitis (*e.g.*, hay fever conjunctivitis, *etc*.), chronic conjunctivitis, trachoma (*e.g.*, granulomatous conjunctivitis, Egyptian ophthalmia, *etc*.), perennial allergic conjunctivitis (*e.g.*, atopic conjunctivitis, atopic keratoconjunctivitis, *etc.*), spring catarrh, episcleritis, scleritis, cicatricial pemphigoid (*e.g.*, ophthal cicatricial pemphigoid, benign mucosal pemphigoid, *etc.*), *etc*.], corneal diseases [*e.g.*, superficial punctate keratitis, corneal ulcer, herpes simplex keratitis (*e.g.*, herpes simplex keratoconjunctivitis, *etc.*), herpes zoster keratitis (*e.g.*, eye herpes zoster, herpes zoster viral ocular symptom, varicella zoster viral ocular symptom, *etc.*), dry keratoconjunctivitis (dry eye) (*e.g.*, dry keratitis, *etc.*), phlyctenular keratoconjunctivitis (*e.g.*, phlyetenular keratitis, *etc.*), interstitial keratitis (*e.g.*, parenchymatous keratitis, *etc.*), marginal ulcerative keratitis (*e.g.*, marginal corneal lysis, marginal rheumatoid ulcer, *etc*.), corneal malacia (*e.g.*, dry keratitis, dry eyeball, *etc.*), keratoconus, *etc*.], cataract, uveitis (*e*.*g*., uveitis syndrome, masquerade syndrome, *etc*.), retinal disorders [*e.g.*, retinal vascular disorder (*e*.*g*., retinal vascular occlusion, ophthalmic hypertension, arteriosclerotic retinopathy, vasospastic retinopathy, renal retinopathy, hypertensive optic nerve retinopathy, hypertensive retinopathy, diabetic retinopathy, central retinal artery occlusion, central retinal vein occlusion, *etc*.), age-related macular degeneration (age-related maculopathy), macular edema, retinal detachment, retinitis pigmentosa, *etc*.], glaucoma, optic nerve/visual pathway disorders (*e*.*g*., choked disc, papillitis, optic nerve papillitis, retrobulbar neuritis, toxic amblyopia, optic atrophy, higher visual pathway lesion, *etc*.), optic neuropathy [*e.g.*, idiopathic optic neuritis, ischemic optic neuropathy, traumatic optic neuropathy, inherited optic neuropathy (*e.g.*, Leber's disease, dominant inherited juvenile optic atrophy, *etc*.), toxic optic neuropathy (*e*.*g*., optic neuropathy induced by an agent for treatment such as chloramphenicol, tetracycline, streptomycin, ethambutol, isoniazid, PAS calcium, a barbituric acid derivative, a digitalis preparation, disulfiram, cisplatin, vincristine, and the like or chemicals such as methyl alcohol, an organic solvent (*e.g.*, toluene, *etc*.) and the like, an organophosphorus pesticide, a carbamate pesticide, *etc*.), syphilitic optic neuritis, tumorous optic neuropathy, *etc.*], neuro-ophthalmologic disorders (*e.g.*, Horner's syndrome, internuclear opthalmoplegia, gaze palsy, *etc*.), amaurosis (*e*.*g*., amaurosis fugax, *etc*.), muscae volitantes, myopia, asthenopia and various symptoms associating these diseases (*e.g.*, disorders of vision or the visual field (*e*.*g*., failing vision, visual field constriction, scotoma in visual field, *etc*.), photophobia, pain, dyschromatopsia, refractive error, *etc*.), and the like. It is preferable that "the agent for the prevention, treatment and/or inhibition of progression of an ophthalmic disease which comprises (2R)-2-propyloctanoic acid, a salt thereof or a prodrug thereof and is intended to be administered at 0.3 ng to 5000 mg per dose" according to the present invention (hereinafter sometimes referred to be simply as "the agent according to the present invention") aims at treating an ophthalmic disease associated with optic nerve disorder among the ophthalmic diseases described above. More specifically speaking, it is preferably used for glaucoma, cataract, retinal detachment, muscae volitantes, age-related macular degeneration, diabetic retinopathy, macular edema, myopia, asthenopia, dry eye, amaurosis fugax, choked disc, papillitis optica, retrobulbar neuritis, toxic amblyopia, optic atrophy, higher visual pathway lesion, internuclear ophthalmoplegia, gaze play and ischemic optic neuropathy and various symptoms associating these diseases. Among all, it is preferable to use the agent according to the present invention for glaucoma, diabetic retinopathy, age-related macular degeneration and the above-described symptoms associating therewith, in particular, disorders in vision and visual field.

In the present invention, glaucoma includes any diseases generally diagnosed as glaucoma. Examples thereof include, for example, primary glaucoma [*e*.*g*., primary open angle glaucoma (*e.g.*, high intraocular pressure glaucoma, normal intraocular pressure glaucoma, *etc.*), primary closed angle glaucoma (*e.g.*, acute closed angle glaucoma, subacute closed angle glaucoma, chronic closed angle glaucoma, plateau iris glaucoma, *etc*.), mixed glaucoma, *etc.*], secondary glaucoma [*e.g.*, secondary open-angle glaucoma (*e.g.*, secondary open angle glaucoma having the principal focus of aqueous humor flow resistance between trabecular meshwork and anterior chamber (*e*.*g*., neovascular glaucoma, glaucoma caused by heterochromia iridis iridocyclitis, glaucoma caused by anterior chamber epithelium proliferation, *etc.*), secondary open angle glaucoma having the principal focus of aqueous humor flow resistance in trabecular meshwork (*e*.*g*., steroid glaucoma, exfoliation glaucoma, glaucoma associating primary amyloidosis, glaucoma caused by uveitis, glaucoma caused by crystalline lens, glaucoma caused by trauma, glaucoma following vitreous surgery, foamy cell glaucoma (ghost cell glaucoma), glaucoma following cataract surgery, glaucoma following corneal transplantation, glaucoma caused by intraocular foreign matters, glaucoma caused by intraocular tumor, Schwartz's syndrome, pigmentary glaucoma, pigment dispersion syndrome, *etc*.), secondary open-angle glaucoma having the principal focus of aqueous humor flow resistance posterior to canal of Schlemm (*e.g.*, glaucoma associating proptosis, glaucoma caused by increase in pressure in the superior vena cava, *etc*.), secondary open angle glaucoma caused by over secretion of aqueous humor, *etc.*), secondary closed angle glaucoma (*e.g.*, secondary closed angle glaucoma caused by pupillary block (*e*.*g*., glaucoma caused by protrusion crystalline lens, glaucoma associating microphthalmos, glaucoma caused by posterior iris synechia, glaucoma caused by lens luxation, glaucoma caused by anterior chamber epithelium proliferation, *etc.*), secondary closed-angle glaucoma caused by the forward migration of a tissue located posterior to crystalline lens (*e.g.*, malignant glaucoma, glaucoma following retinal photocoagulation, glaucoma following selera-shortening surgery, glaucoma caused by intraocular tumor, glaucoma caused by posterior scleritis/Harada's disease, glaucoma caused by central retinal vein occlusion, glaucoma caused by intraocular fillers, glaucoma caused by vitreous mass hemorrhage, *etc.*), secondary closed-angle glaucoma caused by angle synechia which is not associated with pupillary block or migration of crystalline lens diaphragm (*e.g.*, glaucoma following disappearance of anterior chamber or shallow anterior chamber, glaucoma caused by uveitis, glaucoma following corneal transplantation, neovascular glaucoma, ICE syndrome, glaucoma associating iridoschisis, *etc*.), *etc*.), *etc*.], developmental glaucoma [*e*.*g*., early-onset developmental glaucoma, late-onset developmental glaucoma, developmental glaucoma accompanied by other congenital anomaly (*e.g.*, aniridia, Sturge-Weber's syndrome, Axenfeld-Rieger's syndrome, Peter's anomaly, Marfan's syndrome, Weill-Marchesani's syndrome, homocystinuria, neurofibromatosis, rubella syndrome, Pierre Robin's syndrome, chromosomal disorder, persistent hyperplastic primary vitreous, congenital microcornea, Lowe's syndrome, Rubinstein-Taybi's syndrome, Hallermann-Streiff's syndrome, ectropion uveae, *etc*.), *etc*.], secondary glaucoma in infants (*e*.*g*., glaucoma caused by retinopathy of prematurity, glaucoma caused by retinoblastoma, glaucoma caused by juvenile xanthogranuloma, *etc.*), and the like.

In the present invention, diabetic retinopathy includes any conditions having retinal injury caused by diabetes. It may belong to any one of the categories of simple retinopathy, preproliferative retinopathy and proliferative retinopathy. Simple retinopathy means the state where abnormality arises only in retinal vessel, preproliferative retinopathy means the state where an ischemic part (a part with insufficient blood supply) is observed although no neovascularization arises, and proliferative retinopathy means the state where neovascularity is observed and grows toward the vicinity of the vitreous.

To prevent, treat and/or inhibit of symptom progression of an ophthalmic disease, in particular, glaucoma, diabetic retinopathy, age-related macular degeneration, and the like and the above-described symptoms accompanied with the above diseases, the agent of the present invention comprises administering (2R)-2-propyloctanoic acid, a salt thereof or a prodrug thereof is administered at about 0.3 ng to 5000 mg per dose. The administration of (2R)-2-propyloctanoic acid, a salt thereof or a prodrug thereof may be either systemic administration or topical administration. For example, the systemic administration includes that via an oral route (oral administration), a parenteral route (parenteral administration) such as intravenous administration and the like. The topical administration includes, for example, ocular instillation and the like. By changing the administration method as described above, the dose required for achieving a desirable effect changes. For example, in the case of orally administering (2R)-2-propyloctanoic acid, a salt thereof or a prodrug thereof the amount per dose of (2R)-2-propyloctanoic acid, a salt thereof or a prodrug thereof is preferably from about 100 mg to about 5000 mg, more preferably from about 300 mg to about 1200 mg. In the case of intravenously administering (2R)-2-propyloctanoic acid, a salt thereof or a prodrug thereof, the amount per dose of (2R)-2-propyloctanoic acid, a salt thereof or a prodrug thereof is preferably from about 100 mg to about 1200 mg. Furthermore, for example, in the case of administering (2R)-2-propyloctanoic acid, a salt thereof or a prodrug thereof by ocular instillation, the amount per dose of (2R)-2-propyloctanoic acid, a salt thereof or a prodrug thereof is preferably from about 0,3 ng to 1 mg, more preferably from about 30 ng to 300 µg and particularly preferably from about 3 µg to 50 µg.

In the case of intravenously administering (2R)-2-propyloctanoic acid, a salt thereof or a prodrug thereof, it is preferable to define the dose thereof based on the body weight of a mammal (preferably a human being and more preferably a patient) to obtain a preferable treatment effect on the diseases as described above. In the case of a human patient, for example, the administration dose preferably ranges from about 2 mg to about 12 mg per kg of the body weight of the patient. As a more specific example of the administration dose, there can be enumerated about 2 mg, about 4 mg, about 6 mg, about 8 mg, about 10 mg or about 12 mg per kg of the body weight of the patient, and so on. As a more preferable dose, for example, it can be about 4 mg, about 6 mg, about 8 mg or about 10 mg per kg of the body weight of the patient, and the like. As the most preferable dose, for example, it can be about 4 mg or about 8 mg per kg of the body weight of the patient, and the like.

In the case of intravenously administering (2R)-2-propylocianoic acid, a salt thereof or a prodrug thereof, a side effect caused by a rapid increase in the concentration thereof in the blood can be prevented or, if desired, the concentration thereof in the blood can be controlled by continuously administering it into the vein with the use of, for example, an injection cylinder, a transfusion bag, and the like. Although the continuous administration period is not particularly limited and may be altered depending on the conditions of a mammal (a human being is preferable and a patient is more preferable) or other reasons. For example, it is administered continuously from about 0.5 hour to 3 hours, preferably from about 0.5 hour to 1.5 hours and particularly preferably for about 1 hour.

When (2R)-2-prapyloctarzoie acid, a salt thereof or a prodrug thereof to a mammal (a human being is preferable and a patient is more preferable) are administered by using the administration method as described above, a medicinal composition which is appropriate for each administration method is used.

For example, a medicinal composition to be used in the administration by injection such as intravenous administration, and the like such as a so-called transfusion preparation, an injection, and the like can be produced by dissolving (2R)-2-prapyloctazzoic acid, a salt thereof or a prodrug thereof in a solvent such as distilled water for injection, and the like with a metal salt which is commonly used in injections (*e*.*g*., trisodium phosphate, disodium monohydrogen phosphate, sodium carbonate, sodium sulfite, *etc*.), a pH adjusting agent (*e.g.,* sodium hydroxide, *etc*.) and additives described in, for example, lyakuhin Tenkabutsu Jiten, ed. by Nippon lyakuhin Tenkazai Kyokai, Yakuji Nipposha (2000), and the like such as a stabilizer, a surfactant, a buffering agent, a solubilizer, an antioxidant, a defoaming agent, an isotonic agent, an emulsifier, a suspending agent, a preservative, a soothing agent, a dissolving agent, a dissolving aid, and the like. In the case of a transfusion preparation, the components generally used in transfusions such as an electrolyte (*e.g.*, sodium chloride, potassium chloride, calcium chloride, sodium lactate, sodium dihydrogen phosphate, sodium carbonate, magnesium carbonate, *etc*.), a sugar (*e.g.*, glucose, fructose, sorbitol, mannitol, dextran, *etc*.), a proteinous amino acid (*e*.*g*., glycine, aspartic acid, lysine, *etc*.), a vitamin (*e*.*g*., vitamin B₁, vitamin C, *etc*.), and the like may be used in addition to the additives as cited above. Such a medicinal composition is sterilized in the final step or produced ot prepared by an aseptic operation method. It is also possible that an aseptic solid agent such as a freeze-dried product is produced and dissolved in sterilized or aseptic purified water or another solvent before using.

Further for example, a medical composition to be used in ocular instillation (a so-called eye lotion) can be produced by dissolving, suspending or emulsifying (2R)-2-propyloctanoic acid, a salt thereof or a prodrug thereof in a solvent. As the solvent, for example, sterilized and purified water, physiological saline, other aqueous solvents or non-aqueous solvents for injections such as a vegetable oil, and the like, and a combination thereof are used. The eye lotion thus produced may be in any form and it may be a liquid commonly used or a semisolid such as a gel, and the like. If necessary, an eye lotion may further contain an isotonic agent (*e.g.*, sodium chloride, cone. glycerol, *etc*.), a buffering agent (*e*.*g*., sodium phosphate, sodium acetate, *etc*.), a surfactant (*e.g.*, POLYSORBATE 80 (trade name), polyoxyl 40 stearate, polyoxylene-hardened castor oil, *etc*.), a stabilizer (*e.g.*, sodium citrate, sodium edetate, *etc*.), a preservative (*e.g.*, benzalkonium chloride, paraben, *etc.*), and the like. Such a medical composition is sterilized in the final step produced or prepared by an aseptic operation method.

Further for example, a composition to be used in oral administration, namely, a so-called oral preparation, may be in any dosage form so long as it can be orally administered to a mammal (a human being is preferable and a patient having an ophthalmic disease or a need for the protection of optic nerve is more preferable). As an oral preparation containing (2R)-2-propyloctanoic acid, a salt thereof or a prodrug thereof which is used in the present invention is, for example, a tablet, a capsule, subtle granules, granules, a powder, and the like are preferable. A capsule is preferable and a soft capsule is more preferable. For example, a preparation such as a tablet, subtle granules, granules, a powder, and the like can be produced by using a filler, a binder, a disintegrating agent, a lubricant, and the like which are commonly used. Examples of the filler include sucrose, lactose, glucose, starch, mannitol, sorbitol, cellulose, talc, cyclodextrin, and the like. Examples of the binder include cellulose, methylcellulose, polyvinylpyrrolidone, gelatin, gum arabic, polyethylene glycol, sucrose, starch, and the like. Examples of the disintegrating agent include starch, carboxymethylcellulose, carboxymethylcellulose calcium salt, and the like. Examples of the lubricant include talc, and the like. A soft capsule can be produced by, for example, coating (2R)-2-propyloctanoic acid, a salt thereof or a prodrug thereof with a capsule coating commonly used. A capsule coating can be produced by using a capsule base and a plasticizer as the essential components and, if necessary, a flavoring agent, a preservative, a coloring agent, an opacifying agent, a solubility-controlling agent, and the like. Examples of the capsule base include a protein (*e.g.*, gelatin, collagen, *etc*.), a polysaccharide (*e*.*g*., starch, amylose, polygalacturonic acid, agar, carrageenan, acacia, gum arabic, xanthan gum, pectin, alginic acid, *etc.*), a biodegradable plastic (*e.g.*, polylactic acid, polyhydroxybutyric acid, polyglutamic acid, *etc.*), a hardened fat (*e.g.*, triglyceride or diglyceride of medium chain fatty acids, *etc*.), and the like. As the plasticizer, a sugar, a sugar alcohol, a polyhydric alcohol, and the like can be used and examples thereof include glycerol, sorbitol, polyethylene glycol, and the like, Examples of the flavoring agent include peppermint oil, cinnamon oil, essence or flavor of a fruit such as strawberry, and the like. Examples of the preservative include ethyl parahydroxybenzoate, propyl parahydroxybenzoate, and the like. Examples of the coloring agent include Yellow No.4, Yellow No.5, Red No.3, Blue No.1, copper chlorofine, and the like. Examples of the opacifying agent include titanium dioxide, colcothar, and the like. Examples of the solubility-controlling agent include cellulose acetate phthalate, an alkali metal salt of hydroxypropylmethylcellulose, an alkali metal salt of hydroxymethylcellulose acetate succinate, an alkali alginate, an alkali metal polyacrylate, methylcellulose, carboxymethylcellulose, casein, collagen, agar powder, polyvinyl alcohol, pectin, and the like.

In the case of applying such a medical composition for preventing, treating and/or inhibiting symptom progression of an ophthalmic disease, particularly, glaucoma, diabetic retinopathy, age-related macular degeneration and the above-described symptoms associating therewith, the administration of the medicinal composition can be any of the periods, for example, until the onset of the disease is substantially inhibited in the case of desiring a preventive effect; until the treatment is substantially completed in the case of desiring a treatment effect; or until the progression of the symptom is substantially inhibited in the case of desiring an effect of inhibiting symptom progression. If necessary, it can be administered intermittently with an appropriate drug holiday. In the intermittent administration, it is preferable that the drug holiday is 1 day or longer but not longer than 30 days. For example, it may intermittent administration of every second day, intermittent administration consisting of two administration days and one cessation day, intermittent administration consisting of five continuous administration days and two cessation days, or intermittent administration of the so-called calender system (namely in the case of tablets, being called "calender tablets"). For example, the dosing period of the agent of the present invention in the case of, for example, oral administration is from one day to five years, and the like, preferably from 1 day to 1 year, and the like, more preferably from 1 day to 6 months, and the like, and particularly preferably from 1 day to 2 months, and the like. In the case of intravenous administration, the dosing period is from 1 day to 100 days, and the like, preferably from 1 day to 10 days, and the like, more preferably from 1 day to 7 days, and the like, and particularly preferably for 7 days, and the like. In the case of ocular instillation, the dosing period is from 1 day to 1 year, and the like, preferably from 1 day to 6 months, and the like, more preferably from 1 day to 3 months, and the like, and most preferably from 1 day to 1 month, and the like.

During the dosing period, the dosing time per day is, for example, one to five times, and the like, preferably from one to three times, and the like, more preferably one or two times, and the like, and most preferably one time and the like, in each of the administration forms.

Moreover, the present invention also discloses "an agent for protecting optic nerve comprising (2R)-2-propyloctanoic acid or a salt or a prodrug thereof in which an amount per dose is from 0.3 ng to 5000 mg per dose" (hereinafter sometimes referred to be simply as "the agent for protecting optic nerve of the present invention").

The agent for protecting optic nerve of the present invention means an agent capable of making an optic nerve to retain its normal function. Namely, it protects an optic nerve from injury, denaturation or cell death caused by some reasons. Furthermore, an optic nerve suffering from some functional disorder included in the category of the optic nerve to be protected by the agent for protecting optic nerve of the present invention, even though it is physically normal. The physical or functional disorders in optic nerve are not restricted to those caused by the ophthalmic diseases as described above.

In the present invention, the optic nerve may be any cell so long as it relates to visual signal transfer. Specifically, it include a ganglion cell (a retina ganglion cell), an amacrine cell, a bipolar cell, a horizontal cell, a visual cell, a optic nerve cell and the like.

In the optic nerve protecting agent of the present invention, (2R)-2-propyloctanoic acid, a salt or a prodrug thereof is administered at about from 0.3 ng to about 5000 mg per dose in order to protect optic nerve. As the administration methods and medicinal compositions used for the administration, those which are the same as in the agent of the present invention as described above can be used.

A medical composition comprising (2R)-2-propyloctanoic acid, a salt or a prodrug thereof to be used in the present invention may be used either alone or in a combination with another ophthalmic agent. In the case of the use in combination, it is possible to administer a preparation containing both components. Alternatively, the individual preparations may be separately administered. The separate administration of the individual preparations includes simultaneous administration or time-lag administration. Examples of the ophthalmic agent to be used together are as follows: aciclovir, acitazanolast hydrate, amlexanox, anthranilic acid-ascorbic acid, apraclonidine hydrochloride, atropine sulfate, azulene, befunolol hydrochloride, betamethasotie sodium phosphate, betaxolol hydrochloride, boric acid, boric acid-inorganic salts combined drug, brinzolamide, bromfenac sodium hydrate, bunazosin hydrochloride, carteolol hydrochloride, cefmenoxime hydrochloride, chloramphenicol, chloramphemcol-colistin, chondroitin sulfate sodium, chondroitin sulfate sodium-naphazoline hydrochloride, colistin-tetracycline, cyanocobalamin, cyclopentorate hydrochloride cyclopentolate hydrochloride, dexamethasone, dexamethasone metasulfobenzoate sodium, dexamethasone sodium phosphate, dibekacin sulfate, diclofenac sodium, diclofenamide, dipivefrine hydrochloride, distigmine bromide, dorzolamide hydrochloride, erythromycin, erythromycin-colistin, flavin adenine dinucleotide, flavin adenine dinucleotide sodium-chondroitin sulfate sodium, fluorometholone, fradiomycin-betamethasone phosphate, fradiomyein-methylprednisolone, gentamicin sulfate, glucose-inorganic salts combined drug, glutathione, glycyrrhizinate dipotassium, helenien, homatropine hydrobramide, hyaluronate sodium, hyaluronate sodium-chondroitin sulfate sodium, hydrocortisone acetate, ibudilast, idoxuridine, indometacin, isopropyl unoprostone, ketotifen fumarate, latanoprost, levobunolol hydrochloride, levocabastine hydrochloride, levofloxacin, lomefloxacin hydrochloride, lysozyme hydrochloride, methylcellulose, micronomicin sulfate, naphazoline nitrate, neostigmine-inorganic salts combined drug, nipradilol, norfloxacin, ofloxacin, oxiglutatione, oxybuprocaine hydrochloride, oxytetracycline hydrochloride-hydrocortisone, oxytetracycline hydrochloride-polymixin B sulfate, pemirolast potassium, phenylephrine hydrochloride, pilocarpine hydrochloride, pimaricin, pirenoxine, polyvinylalcohol iodine, pranoprofen, prednisolone, prednisolone acetate, silver nitrate, sisomicin sulfate, sodium borate, sodium cromoglicate, sulfisoxazole, timolol maleate, tobramycin, tranilast, tropicamide, tropicamide-phenylephrine hydrochloride, zinc sulfate.

Particularly, examples of other agent which can be used when a medical composition comprising (2R)-2-propyloctanoic acid, a salt thereof or prodrug thereof is used for glaucoma include, for example, sympathomimetic agents (*e.g.*, an α2-receptor agonist such as apraclonidine hydrochloride, a β2-receptor agonist such as dipivefrine hydrochloride, *etc.*), parasympathomimetic agents (*e.g.*, pilocarpine hydrochloride, carbachol, distigmine bromide, *etc*.), sympathetic blockers (*e.g.,* an α1-receptor blocker such as bunazosin hydrochloride and the like, a β-receptor blocker such as timolol maleate, befunolol hydrochloride, carteolol hydrochloride, betaxolol hydrochloride, and the like, an α1β-receptor blocker such as levobunolol hydrochloride, nipradilol, *etc.*), prostaglandins (*e.g.*, isopropyl unoprostone, latanoprost, an EP agonist, an EP2 agonist, an EP4 agonist, a DP agonist, *etc*.), carbonic anhydrase inhibitors (*e*.*g*., acetazolamide, diclofenamide, metazolamide, dorzolamide hydrochloride, brinzolamide, *etc*.), hypertonic osmotic agents (*e.g.*, glycerol, glycerol-fructose preparation, isosorbide, D-mannitol, *etc.*), and the like.

These agents used in combination with a medical composition comprising (2R)-2-propyloctanoic acid, a salt thereof or prodrug thereof are only exemplary and are not limited thereto. The administration methods of these agents are not particularly limited, they may be oral administration or parenteral administration. Also, these agents may be administered in any combination of two or more. Furthermore, the agents for combination use include those that have been discovered as well as those that are to be discovered afterward, based on the mechanism described above.

### Toxicity

Since toxicity of (2R)-2-propyloctanoic acid, a salt thereof or prodrug thereof is low, it can be considered to be sufficiently safe for medicinal uses. For example, in a single intravenous administration test in which (2R)-2-propyloctanoic acid was administered in a dose of 100 mg/kg to dogs, for example, no fatal case was observed.

### Application to Medicament

The present invention comprises administering (2R)-2-propyloctanoic acid, a salt thereof or prodrug thereof for preventing, treating and/or inhibiting symptom progression of an ophthalmic disease and, moreover, for protecting optic nerve in which an amount of per dose is from 0.3 ng to 5000 mg. A medical composition comprising (2R)-2-propyloctanoic acid, a salt thereof or prodrug thereof to be used in the present invention comprises (2R)-2-propyloctanoic acid, a salt thereof or prodrug thereof as the active ingredient and can be used for achieving the above-described object in a mammal (*e.g.*, human being, a non-human animal such as monkey, sheep, bovine, horse, dog, cat, rabbit, rat, mouse, *etc*.). Particularly, by systematically administering via an oral or parenteral route by a preferable method with a preferable dose as disclosed by the present invention to a mammal (a human being is preferable), or by topically administering by, such as ocular instillation, a preferable optic nerve-protecting effect can be shown and preferable effects on an ophthalmic disease can be obtained.

### EXAMPLES

Although the present invention will be described in greater detail by referring to the following EXAMPLES, the present invention is not limited thereto.

It has been proved by, for example, the following experiments that (2R)-2-propyloctanoic acid, a salt thereof or prodrug thereof has an effect of inhibiting cell death caused by retinal ischemia, Measurement methods for evaluating (2R)-2-propyloctanoic acid, a salt thereof or prodrug thereof were modified as follows so as to improve the measurement accuracy and/or measurement sensitivity. Detailed experiment methods are shown as follows:

### EXAMPLE 1: Evaluation of the efficacy of (2R)-2-propyloctanoic acid against nerve cell death in rat retinal ischemia model

### (1) Placement of injection cannula into anterior chamber

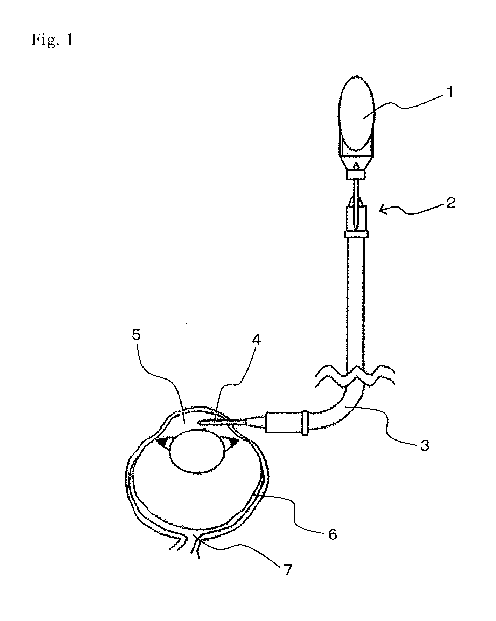

As shown in Fig. 1, to a bottle (1) of an intraocular perfusion solution for ophthalmic surgery (BSS PLUS (trade name); manufactured by SANTEN PHARMACEUTICAL Co., Ltd.), an infant transfusion set (TK-A251 CK002; manufactured by TERUMO CORPORATION) (2) and a 50 cm extension tube (SF-ET3825; manufactured by TERUMO CORPORATION) (3) were connected. A 27G injection needle (4) was attached to an end of the tube. A part about 20 cm below the bottom end of the perfusion line was attached to a supporting bar and the part was lightly fixed by a stand.

### (2) Procedure of loading intraocular pressure (ischemia)

A rat (Crj:CD(SD) IGS, from CHARLES RIVER LABORATORIES JAPAN, Inc.) was anesthetized with pentobarbital sodium (40 mg/kg, i.p.) and the pupil of the eye to be operated were preliminarily dilated by using a mydriasis agent (MYDRIN P; manufactured by SANTEN PHARMACEUTICAL Co., Ltd.).

Before the surgical operation, the bottle (1) was held up so that the water surface in the perfusion bottle was located on 163.5 cm above the rat eyes in the state of closing the perfusion line (loading an intraocular pressure of about 110 mmHg). After opening the top end, the perfusion line was connected to a differential pressure transducer and the height was adjusted to give a predetermined pressure, followed by closing the line. With confirming the mydriasis under a stereoscopic microscope, the stopper of the perfusion line was loosened and the solution was dropped little by little from the needle point. As the cornea was tightly sandwiched between tweezers and an injection needle (4), the injection needle (4) was punctured into the anterior chamber (5) from the nasal side. Next, the perfusion line was opened to load pressure in the anterior chamber (5). After loading the ischemia for 60 minutes, the injection needle (4) was taken off and the animals were awaken out of the anesthesia with keeping the body temperature at 37°C. In the sham-ischemia group, an injection needle was punctured into the anterior chamber from the nasal side in the state of closing the perfusion line, followed by taking off after 60 minutes.

In the figure, (6) stands for the retina while (7) stands for the optic nerve.

### (3) Administration of agent

(2R)-2-propyloctanoic acid was intravenously administered via the tail vein in a dose of 10 mg/mL/kg once a day for 7 days from the day of the ischemic operation. Dizocilpine maleate ((+)-MK-801 maleate, hereinafter referred to simply as "MK-801") which is a positive control compound was intravenously administered via the tail vein in a dose of 10 mg/mL/kg once alone 30 minutes before the ischemic operation.

### (4) Fixation of eye ball

A rat was anesthetized with pentobarbital sodium (40 mg/kg, i.p.) and fixed at the dorsal position. After incising the subcostal skin, with grasping the xiphoid process with tweezers or a kocher, the upper end of the anterior abdominal muscle was horizontally incised with scissors. Next, after reversing the chest wall, the xiphoid process was grasped with a kocher or the chest wall was grasped with a kocher to expose the heart. The heart was grasped with tweezers and the left ventricular was incised with scissors. Then, the front end of a disposable oral probe for mouse connected to a blood transfusion tube was introduced from the left ventricular to the aorta and the end of the oral probe was grasped with a clamp. The atrial auricula was incised and perfused with physiological saline containing heparin (10 U/mL). The blood transfusion tube and the disposable oral probe for mouse were replaced with fresh ones and perfusion with a 10% neutral formalin buffer was conducted in the same manner as described above for fixation. After fixing, both eyes were taken out, stored in a sample bottle filled with the 10% neutral formalin buffer and subjected to the following evaluation.

### (5) Preparation of pathological tissue sample

### (5-1) Preparation of section and staining

An ischemic eye was excised, embedded and sliced. Next, hematoxylin-eosin staining, GFAP (glial fibrillary acidic protein) staining and S100 staining by the methods as shown in the following (5-2) to (5-4) were carried out.

### (5-2) Hematoxylin-eosin staining

### (5-2-a) Reagents used

Hematoxylin (manufactured by MERCK), potassium alum (KARIMYOBAN; manufactured by WAKO PURE CHEMICAL Co., Ltd.), sodium iodate (manufactured by WAKO PURE CHEMICAL Co., Ltd.), chloral hydrate (manufactured by WAKO PURE CHEMICAL Co., Ltd.), crystalline citric acid (manufactured by WAKO PURE CHEMICAL Co., Ltd.), eosin (manufactured by MERCK), pure alcohol (manufactured by MUTO PURE CHEMICAL Co., Ltd.), acetic acid (manufactured by WAKO PURE CHEMICAL Co., Ltd.) and distilled water (manufactured by WAKO PURE CHEMICAL Co., Ltd.) were used.

### (5-2-b) Reagent preparation

### (S-2-b-1) Meyer's hematoxylin solution

Hematoxylin (1.5 g) was dissolved in 10 mL of pure alcohol and distilled water was added to give the total volume of 100 mL (a). Separately, potassium alum (50 g) was added to 700 ml of distilled water and dissolved therein with heating (b). After the dissolution, the hematoxylin solution (a) was added to (b). Next, sodium iodate (0.2 g), chloral hydrate (50 g) and crystalline citric acid (1.0 g) were added thereto and dissolved. Then, the total volume was adjusted to 1000 mL with distilled water.

### (5-2-b-2) Eosin solution

To 100 mL of distilled water, eosin (1 g) and acetic acid (1 mL) were added to give a stock solution. Next, it was diluted 5-fold with 80% alcohol.

### (5-2-c) Staining procedure

Staining was conducted in the following manner:
(1) deparaffinization; (2) water washing; (3) nuclear staining with hematoxylin for 20 minutes; (4) water washing and color development for 10 minutes; (5) eosin for 3 minutes; and (6) dehydration → penetration → enclosure.

### (5-3) GFAP staining

### (5-3-a) Reagent used

An anti-GFAP rabbit antibody (cat No. Z0334, DAKO) was used.

### (5-3-b) GFAP staining procedure

Staining was conducted in the following manner;
(1) preparation of paraffin section → deparaffinization; (2) endogenous peroxidase block at room temperature for 10 min; (3) washing with running water; (4) bovine albumin at 37°C for 10 minutes; (5) anti-GFAP rabbit antibody (diluted 250-fold) at 37°C for 60 minutes; (6) washing with TBS buffer; (7) biotin-labeled anti-rabbit Ig goat antibody (diluted 500-fold) at 37°C for 30 minutes; (8) washing with TBS buffer; (9) peroxidase-labeled streptavidin (diluted 500-fold) at 37°C for 30 minutes; (10) washing with TBS buffer; (11) DAB coloration reaction; (12) washing with running water; (13) hematoxylin for 10 minutes; (14) dehydration → penetration → enclosure.

### (5-4) S100 staining

### (5-4-a) Reagent used

An anti-S100 rabbit antibody (cat No. Z0311, DAKO) was used.

### (5-4-b) S100 staining procedure

Staining was conducted in the following manner:
(1) preparation of paraffin section → deparaffinization; (2) endogenous peroxidase block at room temperature for 10 minutes; (3) washing with running water; (4) bovine albumin at 37°C for 10 minutes; (5) anti-S100 rabbit antibody (diluted 250-fold) at 37°C for 60 minutes; (6) washing with TBS buffer; (7) biotin-labeled anti-rabbit Ig goat antibody (diluted 500-fold) at 37°C for 30 minutes; (8) washing with TBS buffer; (9) peroxidase-labeled streptavidin (diluted 500-fold) at 37°C for 30 minutes; (10) washing with TBS buffer; (11) DAB coloration reaction; (12) washing with running water; (13) hematoxylin for 10 minutes; (14) dehydration → penetration → enclosure.

### (6) Pathological tissue evaluation and results thereof

### (6-1) Evaluation of number of GCL cells

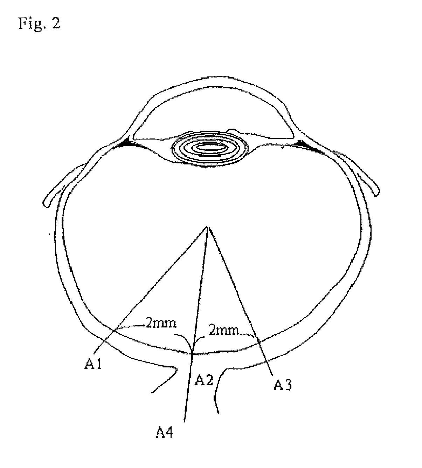

Using HE-stained samples thus obtained, GCL cells at two points (A1, A3; refer to Fig. 2) on the retina which are 2 mm apart from the optic nerve papplila (A2) were counted (cells/mm²).

### <Results>

The results are shown in Table 1.

Against the decrease in the number of optic nerve cells caused by the ischemia load, (2R)-2-propyloctanoic acid and MK-801 both showed inhibitory effects (Table 1). The inhibitory rate in the sum of the A1 and A3 data (A1+A3) of (2R)-2-propyloctanoic acid was 52.1%, while that of MK-801 was 59.5%.

**Table 1**

| Group | Dose (mg/kg) | No. of animals | | Number of GCL cells | | |
|---|---|---|---|---|---|---|
| | | | | A1 | A3 | A1+A3 |
| Control | - | 8 | Mean | 162.5 | 172.6 | 335.1 |
| | | | S.D. | 16.7 | 18.0 | 16.5 |
| Injury control | - | 7 | Mean | 69.1 | 80.4 | 149.6 |
| | | | S.D. | 29.0 | 37.0 | 63.1 |
| (2R)-2-propyloctanic acid | 10 | 7 | Mean | 116.4 | 129.9 | 246.3 |
| | | | S.D. | 41.2 | 31.6 | 71.8 |
| MK-801 | 10 | 7 | Mean | 125.1 | 134.9 | 260.0 |
| | | | S.D. | 42.2 | 44.6 | 82.6 |

### (6-2) Evaluation of number of glia cells and area

By using GFAP-stained samples thus obtained, GFAP-positive cells at A1, A2 and A3 were counted (cells/mm²) and GFAP-positive areas at A1, A2, A3 and A4 (optic nerve; refer to Fig. 2) were measured (% AREA).

### <Results>

The results are shown in Tables 2 and 3.

By loading the ischemia, GFAP-positive cells decreased at the A1 and A3 points and increased at the A2 point. Against these changes in the number of GFAP-positive cells, (2R)-2-propyloctanoic acid and MK-801 both showed inhibitory effects (Table 2). For example, the inhibitory rate at the point A1 of (2R)-2-propyloctanoic acid was 69.0%, while that of MK-801 was 42.9%.

**Table 2**

| Group | Dose (mg/kg) | No. of animals | | Number of GFAP positive cells | | | |
|---|---|---|---|---|---|---|---|
| | | | | A1 | A2 | A3 | A4 |
| Control | - | 8 | Mean | 58.3 | 273.4 | 54.3 | ND |
| | | | S.D. | 20.5 | 112.7 | 8.8 | - |
| Injury control | - | 7 | Mean | 39.9 | 414.9 | 37.1 | ND |
| | | | S.D. | 19.6 | 115.1 | 9.6 | - |
| (2R)-2-propyloctanoic acid | 10 | 7 | Mean | 52.6 | 317.9 | 52.9 | ND |
| | | | S.D. | 17.2 | 128.9 | 19.2 | - |
| MK-801 | 10 | 7 | Mean | 47.8 | 275.3 | 56.8 | ND |
| | | | S.D. | 20.5 | 60.8 | 13.3 | - |

Additionally, the GFAP positive areas increased by loading the ischemia at all of the A1, A2, A3 and A4 points. Both of (2R)-2-propyloctanoic acid and MK-801 showed inhibitory effects on these increases in the GFAP positive areas (Table 3). For example, the inhibitory rate at the point A1 of (2R)-2-propyloctanoic acid was 87.8%, while that of MK-801 was 78.3%.

**Table 3**

| Group | Dose (mg/kg) | No. of animals | | Area of GFAP positive cells | | | |
|---|---|---|---|---|---|---|---|
| | | | | A1 | A2 | A3 | A4 |
| Control | - | 8 | Mean | 1.7 | 10.5 | 1.9 | 15.4 |
| | | | S.D. | 0.6 | 3.1 | 0.9 | 6.1 |
| Injury control | - | 7 | Mean | 5.1 | 14.3 | 5.0 | 26.9 |
| | | | S.D. | 2.7 | 4.4 | 2.7 | 4.4 |
| (2R)-2-propyloctanoic acid | 10 | 7 | Mean | 4.2 | 10.1 | 4.2 | 16.8 |
| | | | S.D. | 1.2 | 2.4 | 2.2 | 8.1 |
| MK-801 | 10 | 7 | Mean | 3.4 | 11.0 | 3.6 | 17.9 |
| | | | S.D. | 1.4 | 3.4 | 1.5 | 4.6 |

### (6-3) Evaluation of number of S100-positive cells and area

Using S100-stained samples thus obtained, S100-positive cells at A2 and A4 were counted (cells/mm²) and S100-positive areas at A1, A2, A3 and A4 (% AREA) were measured.

### <Results>

The results are shown in Tables 4 and 5.

By loading the ischemia, S100-positive cells increased at the A2 and A4 points. Against these changes in the number of S-100-positive cells, (2R)-2-propyloctanoic acid showed an inhibitory effect (Table 4). For example, the inhibitory rate at the point A4 of (2R)-2-propyloctanoic acid was 31.2%. On the other hand, MK-801 showed an inhibitory effect exclusively on the increase in the number of S100-positive cells at the A2 point.

**Table 4**

| Group | Dose (mg/kg) | No. of animals | | Number of S100 positive cells | | | |
|---|---|---|---|---|---|---|---|
| | | | | A1 | A2 | A3 | A4 |
| Control | - | 8 | Mean | ND | 339.5 | ND | 54.1 |
| | | | S.D. | - | 103.1 | - | 30.7 |
| Injury control | - | 7 | Mean | ND | 487.9 | ND | 422.8 |
| | | | S.D. | - | 195.8 | - | 271.1 |
| (2R)-2-propyloctanoic c acid | 10 | 7 | Mean | ND | 295.4 | ND | 307.7 |
| | | | S.D. | - | 112.6 | - | 212.3 |
| MK-801 | 10 | 7 | Mean | ND | 212.9 | ND | 600.2 |
| | | | S.D. | - | 50.8 | - | 241.0 |

Additionally, the S 100 positive areas remarkably increased by loading the ischemia only at the A4 point. Both of (2R)-2-propyloctanoic acid and MK-801 showed inhibitory effects on these increases in the S100 positive areas (Table 5). For example, the inhibitory rate at the point A4 of (2R)-2-propyloctanoic acid was 93.9%, while that of MK-801 was 51.2%.

**Table 5**

| Group | Dose (mg/kg) | No. of animals | | Area of S100 positive cells | | | |
|---|---|---|---|---|---|---|---|
| | | | | A1 | A2 | A3 | A4 |
| Control | - | 8 | Mean | 1.3 | 2.5 | 1.8 | 2.8 |
| | | | S.D. | 0.4 | 1.3 | 1.0 | 2.6 |
| Injury control | - | 7 | Mean | 1.9 | 4.5 | 1.9 | 11.0 |
| | | | S.D. | 1.5 | 3.0 | 0.7 | 12.7 |
| (2R)-2-propyloctanoic acid | 10 | 7 | Mean | 1.9 | 3.5 | 1.6 | 3.3 |
| | | | S.D. | 1.5 | 1.7 | 1.1 | 5.3 |
| MK-801 | 10 | 7 | Mean | 1.9 | 3.8 | 2.1 | 6.8 |
| | | | S.D. | 1.1 | 2.2 | 1.3 | 6.3 |

### PREPARATION EXAMPLES

### PREPARATION EXAMPLE 1: Production of injection comprising (2R)-2-propyloctanoic acid

To water for injection, (2R)-2-propyloctanoic acid (2.0 kg) and trisodium phosphate 12-hydrate (3.54 kg) were added. The total volume was adjusted to 40 L with the water for injection. After obtaining a homogeneous solution, it was filtered through a sterilizing filter (Durapore 0.22 µm membrane) and packed in plastic ampules in 2 mL portions. After sterilizing with autoclave (123°C, 15 minutes), 20,000 ampules comprising 100 mg of the active ingredient per ampule were obtained.

### PREPARATION EXAMPLE 2: Production of soft capsules containing (2R)-2-propyloctanoic acid

Gelatin (20 kg) and cone. glycerol (6 kg) were blended together in the presence of purified water (20 kg) under 70°C to give a homogeneous solution. The solution and (2R)-2-propyloctanoic acid (0.9 kg) were supplied into a soft capsule encapsulating machine (a rotary soft capsule molding machine Model H-1; manufactured by KAMATA) to give coarse soft capsules having (2R)-2-propyloctanoic acid encapsulated therein. The coarse soft capsules were subjected to a tumbler drying and a tray drying in order to obtain and thus soft capsules (2200 capsules) comprising 300 mg of (2R)-2-propyloctanoic acid per capsule were obtained.

### PREPARATION EXAMPLE 3: Production of eye lotion comprising (2R)-2-propyloctanoic acid

To (2R)-2-propyloctanoic acid (1.0 g), Polysorbate 80 (0.5 g), chlorobutanol (0.5 g), methyl parahydroxybenzoate (0.1 g), ethyl parahydroxybenzoate (0.1 g), butyl parahydroxybenzoate (0.1 g) and sodium hydrogen phosphate (1.0 g) were added and these components were dissolved in sterilized purified water under stirring. Next, sodium hydroxide (an appropriate amount) and dilute hydrochloric acid (an appropriate amount) were added to adjust pH to be 7.0. Thus, an eye lotion (total volume: 100 mL) was obtained.

### INDUSTRIALAPPLICABILITY

The agent for the prevention, treatment and/or inhibition of progression of an ophthalmic disease and the agent for protecting optic nerve comprising (2R)-2-propyloctanoic acid, a salt thereof or a prodrug in which an amount per dose is from 0.3 ng to 5000 mg are safe and can inhibit optic nerve cell death associating ophthalmic diseases such as glaucoma, diabetic retinopathy, age-related macular degeneration, and the like. Therefore, the agents remarkably ameliorate various symptoms accompanied with the above diseases, so they are useful as a medicament.

### BRIEF DESCRIPTION OF THE DRAWINGS

[Fig. 1] Fig. 1 is a drawing which shows the placement of an injection cannula into the anterior chamber.
[Fig. 2] Fig. 2 is a drawing which shows regions for pathological tissue evaluation.

## Claims

1. An agent comprising (2R)-2-propyloctanoic acid or a salt thereof for use in the prevention, treatment and/or inhibition of symptom progression of subject of an ophthalmic disease selected from the group consisting of glaucoma, cataract, retinal detachment, muscle volitantes, age-related macular degeneration, diabetic retinopathy, macular edema, myopia, asthenopia, choked disc, papillitis optica, retrobulbar neuritis, toxic amblyopia, optic atrophy, higher visual pathway lesion, internuclear ophthalmoplegia, gaze palsy and ischemic optic neuropathy, wherein the agent is administered intravenously for from one day to 7 days at a dose of from 2 mg to 12 mg per 1 kg of body weight of a patient, orally in an amount per dose of from 300 mg to 1200 mg for from one day to five years or by ocular instillation in an amount per dose of from 0.3 ng to 1 mg for from one day to one year.

2. The agent for use according to claim 1, wherein the agent is administered intravenously for from one day to 7 days at a dose of from 2 mg to 12 mg per 1 kg of body weight of a patient.

3. The agent for use according to claim 2, wherein the dose per 1 kg of body weight of a patient is 10 mg.

4. The agent for use according to claim 1 or 2, wherein the agent is administered orally in an amount per dose of from 300 mg to 1200 mg for from one day to five years or by ocular instillation in an amount per dose of from 0.3 ng to 1 mg for from one day to one year.

5. The agent for use according to claim 1, wherein the agent is administered orally in an amount per dose of from 300 mg to 1200 mg for from one day to five years.

6. An agent for use in protecting optic nerve comprising (2R)-2-propyloctanoic acid or a salt thereof in which an amount per dose is from 0.3 ng to 5000 mg per dose.

7. The agent for use according to claim 6, wherein the agent is administered intravenously for from one day to 7 days at a dose of from 2 mg to 12 mg per 1 kg of body weight of a patient, orally in an amount per dose of from 300 mg to 1200 mg for from one day to five years or by ocular instillation in an amount per dose of from 0.3 ng to 1 mg for from one day to one year.

8. The agent for use according to claim 1, wherein the ophthalmic disease is glaucoma and the agent is administered intravenously for 7 days at a dose of from 2 mg to 12 mg per 1 kg of body weight of a patient.

9. The agent for use according to claim 1, wherein the ophthalmic disease is glaucoma and the agent is administered intravenously for 7 days at a dose of from 2 mg to 12 mg per 1 kg of body weight of a patient, in the form of the injection which also optionally comprises a solvent, a metal salt, a pH adjusting agent, a stabilizer, a surfactant, a buffering agent, a solubilizer, an antioxidant, a defoaming agent, an isotonic agent, an emulsifier, a suspending agent, a preservative, a soothing agent, a dissolving agent, and/or a dissolving aid.

10. The agent according to claim 9, wherein the dose per I kg of body weight of a patient is 10 mg.

11. The agent for use according to claim 1, wherein the ophthalmic disease is glaucoma and the agent is administered orally in an amount per dose of from 300 mg to 1200 mg for from one day to five years, in the form of the soft capsule which also optionally comprises a filler, a binder, a disintegrating agent and/or a lubricant.

12. The agent for use according to claim 1, wherein the ophthalmic disease is glaucoma and the agent is administered by ocular instillation in an amount per dose of from 0.3 ng to 1 mg for from one day to one year, in the form of the ophthalmic preparation which also optionally comprises a solvent, an isotonic agent, a buffering agent, a surfactant, a stabilizer and/or a preservative.
